# EUROPEAN PATENT SPECIFICATION

(11) **EP 3 681 476 B1**
(45) Date of publication and mention of the grant of the patent: **14.09.2022**
(21) Application number: 18800352.9
(22) Date of filing: 14.09.2018
(51) Int. Cl.: A61K 9/00, A61K 47/08, A61K 47/10, A61K 47/12, A61K 47/22, A61K 9/08, A61K 31/26

(54) **TRANSDERMAL FORMULATIONS**
TRANSDERMALE FORMULIERUNGEN
PRÉPARATIONS TRANSDERMIQUES

(30) Priority: 15.09.2017 US 201762559156 P
(43) Date of publication of application: 22.07.2020
(73) Proprietor: Tyme, Inc., Bedminster, NJ 07921 (US)
(72) Inventor: HOFFMAN, Steven, Mahwah NJ 07430 (US)
(74) Representative: Høiberg P/S
(86) International application number: PCT/US2018/051010
(87) International publication number: WO 2019/055747

(56) References cited:
- EP-A1- 1 961 418
- JP-A- 2004 300 143
- US-A1- 2016 199 453

## Description

### CROSS REFERENCE TO RELATED APPLICATIONS

This application claims the benefit of U.S. Provisional Patent Application No. 62/559,156, filed September 15, 2017.

### BACKGROUND

Transdermal administration of therapeutic agents has many advantages, including convenience and gastrointestinal tract metabolism avoidance. But in the absence of penetration enhancing agents, many therapeutic agents are not capable of penetrating the skin in therapeutically effective concentrations. As such, compositions that facilitate the penetration of therapeutic agents through the skin are needed.

Therapeutic agents that would be advantageously delivered transdermally include isothiocyanates and isocyanates. Such compounds are therapeutically useful due to their anticancer effects. *See*, *e.g*., Wattenberg LW. Inhibition of carcinogen-induced neoplasia by sodium cyanate, tert-butyl isocyanate, and benzyl isothiocyanate administered subsequent to carcinogen exposure. Cancer Research. 1981 Aug 1;41(8):2991-4.; Hecht SS. Chemoprevention of cancer by isothiocyanates, modifiers of carcinogen metabolism. The Journal of nutrition. 1999 Mar 1;129(3):768S-74S; Shapiro TA, Fahey JW, Wade KL, Stephenson KK, Talalay P. Human metabolism and excretion of cancer chemoprotective glucosinolates and isothiocyanates of cruciferous vegetables. Cancer Epidemiology and Prevention Biomarkers. 1998 Dec 1;7(12):1091-100; Gupta P. et al., Phenethyl Isothiocyanate: A comprehensive review of anti-cancer mechanisms, Biochim Biophys Acta. 2014 December; 1846(2): 405-424; Gupta, P. et al. "Molecular Targets of Isothiocyanates in Cancer: Recent Advances. "Molecular nutrition & food research 58.8 (2014): 1685-1707. Patent document EP1961418 discloses compositions comprising isothiocyanates.

In view of the chemotherapeutic and chemoprotective activities of isothiocyanates and isocyanates, compositions that facilitate the penetration of isothiocyanates and isocyanates through the skin are needed.

### SUMMARY

The present disclosure is directed to compositions according to claim 1 comprising a first component, a second component, a C₂₋₁₀alkyl alcohol, and an organic acid having 1 to 25 carbon atoms, and a therapeutic agent, wherein the first and second components and the therapeutic agent are further defined herein. Methods of making and using these compositions are also described.

### DETAILED DESCRIPTION OF ILLUSTRATIVE EMBODIMENTS

The present disclosure may be understood more readily by reference to the following detailed description of desired embodiments and the examples included therein. In the following specification and the claims that follow, reference will be made to a number of terms which have the following meanings.

As used in the specification and in the claims, the term "comprising" may include the embodiments "consisting of" and "consisting essentially of." The terms "comprise(s)," "include(s)," "having," "has," "can," "contain(s)," and variants thereof, as used herein, are intended to be open-ended transitional phrases, terms, or words that require the presence of the named ingredients/steps and permit the presence of other ingredients/steps. However, such description should be construed as also describing compositions or processes as "consisting of" and "consisting essentially of" the enumerated ingredients/steps, which allows the presence of only the named ingredients/steps, along with any impurities that might result therefrom, and excludes other ingredients/steps.

Unless indicated to the contrary, the numerical values should be understood to include numerical values which are the same when reduced to the same number of significant figures and numerical values which differ from the stated value by less than the experimental error of conventional measurement technique of the type described in the present application to determine the value.

All ranges disclosed herein are inclusive of the recited endpoint and independently combinable (for example, the range of "from 2 to 10" is inclusive of the endpoints, 2 and 10, and all the intermediate values). The endpoints of the ranges and any values disclosed herein are not limited to the precise range or value; they are sufficiently imprecise to include values approximating these ranges and/or values.

As used herein, approximating language may be applied to modify any quantitative representation that may vary without resulting in a change in the basic function to which it is related. Accordingly, a value modified by a term or terms, such as "about" and "substantially," may not be limited to the precise value specified, in some cases. In at least some instances, the approximating language may correspond to the precision of an instrument for measuring the value. The modifier "about" should also be considered as disclosing the range defined by the absolute values of the two endpoints. For example, the expression "from about 2 to about 4" also discloses the range "from 2 to 4." The term "about" may refer to plus or minus 10% of the indicated number. For example, "about 10%" may indicate a range of 9% to 11%, and "about 1" may mean from 0.9-1.1. Other meanings of "about" may be apparent from the context, such as rounding off, so, for example "about 1" may also mean from 0.5 to 1.4.

As used herein, "alkyl" refers to straight chain and branched chains having the indicated number of carbon atoms, usually from 1 to 20 carbon atoms, for example 1 to 8 carbon atoms, such as 1 to 6 or 1 to 7 carbon atoms. For example C₁₋₆ alkyl encompasses both straight and branched chain alkyl of from 1 to 6 carbon atoms. When an alkyl residue having a specific number of carbons is named, all branched and straight chain versions having that number of carbons are intended to be encompassed; thus, for example, "butyl" is meant to include n-butyl, sec-butyl, isobutyl and t-butyl; "propyl" includes n-propyl and isopropyl. Examples of alkyl groups include methyl, ethyl, propyl, isopropyl, n-butyl, sec-butyl, tert-butyl, pentyl, 2-pentyl, isopentyl, neopentyl, hexyl, 2-hexyl, 3-hexyl, 3-methylpentyl, and the like. Alkyl groups may be optionally substituted with one or more halogen atoms (F, Cl, Br, or I), hydroxy groups, alkoxy groups, or methylsulfinyl (*i.e*., CH₃-S(O)-) groups.

As used herein, "aryl" when used alone or as part of a substituent group refers to a mono- or bicyclic- aromatic hydrocarbon ring structure having 5 or 10 carbon atoms in the ring, wherein one or more of the carbon atoms in the ring is optionally substituted. Substituent groups may include one or more halogen atoms, -C₁₋₆alkyl groups, or -C₁₋₆alkoxyl groups. Halogen atoms include chlorine, fluorine, bromine, and iodine. C₁₋₆alkoxyl groups include, for example, methoxy, ethoxy, propoxy, and the like. Examples of aryl groups (substituted and unsubstituted) include phenyl, naphtyl, fluorophenyl, difluorophenyl, chlorophenyl, dichlorophenyl, bromophenyl, iodophenyl, chlorofluorophenyl, fluoronaphthyl, difluoronaphthyl, chloronaphthyl, bromonaphthyl, iodonaphthyl, methylphenyl, ethylphenyl, (trifluoromethyl)phenyl, and the like.

The term "C₁-C₆alk" when used alone or as part of a substituent group refers to an aliphatic linker having 1, 2, 3, 4, 5, or 6 carbon atoms and includes, for example, -CH₂-, -CH(CH₃)-, -CH(CH₃)-CH₂-, and -C(CH₃)₂-. In some aspects, the C₁-C₆alk can be substituted with one or more substituents.

As used herein, "alkenyl" refers to an unsaturated branched or straight-chain alkyl group having at least one carbon-carbon double bond. The group may be in either the cis or trans configuration about the double bond(s). The group may also be an aromatic group, for example, a phenyl or phenylene moiety. Typical alkenyl groups include, but are not limited to, ethenyl; propenyls such as prop-1-en-1-yl, prop-1-en-2-yl, prop-2-en-1-yl (allyl), prop-2-en-2-yl; butenyls such as but-1-en-1-yl, but-1-en-2-yl, 2-methyl-prop-1-en-1-yl, but-2-en-1-yl, but-2-en-1-yl, but-2-en-2-yl, buta-1,3-dien-1-yl, buta-1,3-dien-2-yl; phenylene, and the like. In certain embodiments, an alkenyl group has from 2 to 20 carbon atoms.

As used herein, "alkynyl" refers to an unsaturated branched or straight-chain alkyl group having at least one carbon-carbon triple bond derived by the removal of two molecules of hydrogen from adjacent carbon atoms of the parent alkyl. Typical alkynyl groups include, but are not limited to, ethynyl; propynyls such as prop-1-yn-1-yl, prop-2-yn-1-yl; butynyls such as but-1-yn-1-yl, but-1-yn-3-yl, but-3-yn-1-yl; and the like. In certain embodiments, an alkynyl group has from 2 to 20 carbon atoms.

As used herein, the terms "treatment" or "therapy" (as well as different forms thereof) include preventative (e.g., prophylactic), curative or palliative treatment. As used herein, the term "treating" includes alleviating or reducing at least one adverse or negative effect or symptom of a condition, disease or disorder. This condition, disease or disorder can be cancer.

As employed above and throughout the disclosure the term "effective amount" refers to an amount effective, at dosages, and for periods of time necessary, to achieve the desired result with respect to the treatment of the relevant disorder, condition, or side effect. It will be appreciated that the effective amount of components of the present invention will vary from patient to patient not only with the particular compound, component or composition selected, the route of administration, and the ability of the components to elicit a desired result in the individual, but also with factors such as the disease state or severity of the condition to be alleviated, hormone levels, age, sex, weight of the individual, the state of being of the patient, and the severity of the pathological condition being treated, concurrent medication or special diets then being followed by the particular patient, and other factors which those skilled in the art will recognize, with the appropriate dosage being at the discretion of the attending physician. Dosage regimes may be adjusted to provide the improved therapeutic response. An effective amount is also one in which any toxic or detrimental effects of the components are outweighed by the therapeutically beneficial effects.

"Pharmaceutically acceptable" refers to those compounds, materials, compositions, and/or dosage forms which are, within the scope of sound medical judgment, suitable for contact with the tissues of human beings and animals without excessive toxicity, irritation, allergic response, or other problem complications commensurate with a reasonable benefit/risk ratio.

The present disclosure is directed to compositions that facilitate and/or enhance the transdermal permeation of therapeutic agents through the skin. As used herein, the term "transdermal permeation" includes both percutaneous delivery and transmucosal delivery, that is, passage through skin or mucosal tissue and into the bloodstream. As used herein in reference to transdermal penetration, the term "enhancing" refers to increasing the rate at which a therapeutic agent penetrates the skin or mucosal tissue and enters the bloodstream. These compositions include a first component, a second component, an alcohol, an organic acid, a therapeutic agent, and, optionally, water.

According to the disclosure, the first component comprises
- a compound of formula (I):

   R-(OCH₂CH₂)_{y}-OH (I)

   wherein R is C₁₋₂₀alkyl, C₂₋₂₀alkenyl; or C₂₋₂₀alkynyl; and y is 1 to 25;
- a tetrafunctional block copolymer surfactant terminating in primary hydroxyl groups;
- a sorbitan derivative;
- a C₈₋₁₀alkyl ammonium salt;
- a compound of formula (II):

   HO-(CH₂CH₂O)ₘ-C(CH₃)(C₄H₉)-C≡C-C(CH₃)(C₄H₉)-(OCH₂CH₂)ₙ-OH (II)

   wherein m and n are each independently 1 to 25;
or a combination thereof.

In preferred embodiments of the disclosure, the first component is a compound of formula (I). In some embodiments, R is C₁₋₂₀alkyl, which can either be a straight chain or branched alkyl. Preferred compounds of formula (I) wherein R is C₁₋₂₀alkyl include, for example, is cetomacrogol 1000; octadecan-1-ol, ethoxylated; polyoxyethylene(12)tridecyl ether; polyoxyethylene(10)tridecyl ether; fatty alcohol polyoxyethylene ether, polyoxyethylene branched nonylcyclohexyl ether (TRITON N-101), nonaethylene glycol monododecyl ether, 23-{[4-(2,4,4-trimethyl-2-pentanyl)cyclohexyl]oxy}-3,6,9,12,15,18,21-heptaoxatricosan-1-ol, and combinations thereof. Nonaethylene glycol monododecyl ether is particularly preferred.

In other embodiments, R is C₂₋₂₀alkenyl, which can either be a straight chain or branched alkenyl. Preferred compounds of formula (I) wherein R is C₂₋₂₀alkenyl include, for example, polyoxyl(10)oleyl ether, polyethylene glycol tert-octylphenyl ether (TRITON X-100), and combinations thereof

In yet other embodiment, R is C₂₋₂₀alkynyl, which can either be a straight chain or branch alkynyl.

In those embodiments wherein the first component is a compound of formula (I), y is 1 to 25. In preferred embodiments, y is 5 to 15, preferably 8 to 10, with 9 being particularly preferred. In other embodiments, y is 1, 2, 3, 4, 5, 6, 7, 8, 9, 10, 11, 12, 13, 14, 15, 16, 17, 18, 19, 20, 21, 22, 23, 24, or 25.

In other aspects of the disclosure, the first component is a tetrafunctional block copolymer surfactant terminating in primary hydroxyl groups. Such compounds are commercially available under the tradename TETRONIC^{™} and include ethylenediaminetetrakis(ethoxylate-Block-propoxylate).

In other embodiments of the disclosure, the first component is a sorbitan derivative, for example, polyoxyethylene sorbitan tetraoleate, 1,4-anhydro-6-O-palmitoyl-D-glucitol (sorbitan, monohexadecanoate), a polyethylene glycol sorbitan monolaurate (e.g., TWEEN^{™} 20, TWEEN^{™} 40, TWEEN^{™} 60, TWEEN^{™} 85), and combinations thereof.

In still other embodiments of the disclosure, the first component is a C₈₋₁₀alkyl ammonium salt, for example, methyltrialkyl(C₈-C₁₀)ammonium chloride (ADOGEN^{™} 464).

In other embodiments, the first component is a compound of formula (II).

The compositions of the disclosure can comprise from about 0.1 vol.% to about 40 vol.% of the first component. In preferred embodiments, the compositions comprise from about 1 vol.% to about 40 vol.% of the first component. In other embodiments, the compositions comprise from about 0.1 vol.% to about 5 vol.% of the first component. For example, the compositions can comprise about 0.1, 0.2, 0.3, 0.4, 0.5, 0.6, 0.7, 0.8, 0.9, 1, 1.5, 2, 2.5, 3, 3.5, 4, 4.5, 5, 5.5, 6, 6.5, 7, 7.5, 8, 8.5, 9, 9.5, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or about 40 vol.% of the first component.

According to the disclosure, the compositions also include a second component that comprises
- an compound of the formula (III):

   R²-N(R¹)-C(O)-R³ (III)

   wherein each R¹ is independently H or C₁₋₃alkyl; and
   R² and R³ are independently C₁₋₇alkyl or together with the atoms to which they are attached, form a lactam having 3 to 10 carbon atoms,
- a sulfoxide;
- a urea;
- ethyl acetate;
or a combination thereof.

In preferred embodiments, the second component is a compound of formula (III). In some embodiments, R¹ is H. In other embodiments, R¹ is methyl, ethyl, propyl, or isopropyl, with methyl being particularly preferred.

In those embodiments wherein R² and R³ are independently C₁₋₇alkyl, each of R² and R³ is independently methyl, ethyl, propyl, isopropyl, butyl, s-butyl, t-butyl, pentyl, hexyl, or heptyl.

Preferably, R² and R³, together with the atoms to which they are attached, form a lactam having 3 to 10 carbon atoms. For example, the lactam can include 3, 4, 5, 6, 7, 8, 9, or 10 carbons, which can be a part of the lactam ring or which can form exocyclic branching. Examples of preferred lactams include pyrrolidones such as 2-pyrrolidone, 1-methyl-2-pyrrolidone, 5-methyl-2-pyrrolidone, and 1-ethyl-2-pyrrolidone. Preferably, the lactam is 1-methyl-2-pyrrolidone or 2-pyrrolidone.

In some embodiments, the second component is a sulfoxide, for example, dimethyl sulfoxide.

In other embodiments, the second component is a urea, for example an imidazolidinone.

The compositions of the disclosure can comprise from about 0.01 vol.% to about 10 vol.% of the second component. In preferred embodiments, the compositions comprise from about 0.01 vol.% to about 5 vol.% of the second component. In other embodiments, the compositions comprise from about 0.01 vol.% to about 4 vol.% of the second component. For example, the compositions can comprise about 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1, 1.5, 2, 2.5, 3, 3.5, 4, 4.5, 5, 5.5, 6, 6.5, 7, 7.5, 8, 8.5, 9, 9.5, or about 10 vol.% of the second component.

In some embodiments of the disclosure, the ratio, by volume, of the first component to the second component is about 10:1 to about 4:1.

According to the disclosure, the compositions also include a C₂₋₁₀ alkyl alcohol. Alcohols for use in the compositions of the disclosure include C₂₋₁₀alkyl alcohols having at least one -OH moiety or at least two -OH moieties. For example, preferred alcohols include glycerol, propylene glycol, ethanol, isopropanol, 1-propanol, butanol, t-butanol, pentanol, 1-octanol, and combinations thereof, with ethanol being particularly preferred.

The compositions of the disclosure can comprise from about 0.1 vol.% to about 50 vol.% of the C₂₋₁₀ alkyl alcohol. In preferred embodiments, the compositions comprise from about 1 vol.% to about 50 vol.% of the C₂₋₁₀ alkyl alcohol. In other embodiments, the compositions comprise from about 0.1 vol.% to about 5 vol.% of the C₂₋₁₀ alkyl alcohol. For example, the compositions can comprise about 0.1, 0.2, 0.3, 0.4, 0.5, 0.6, 0.7, 0.8, 0.9, 1, 1.5, 2, 2.5, 3, 3.5, 4, 4.5, 5, 5.5, 6, 6.5, 7, 7.5, 8, 8.5, 9, 9.5, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or about 50 vol.% of the C₂₋₁₀ alkyl alcohol.

According to the disclosure, the compositions also include an organic acid having 1 to 25 carbon atoms. For example, organic acids for use in the disclose compositions include acetic acid, ascorbic acid, lactic acid, glycolic acid, propionic acid, and combinations thereof.

Other organic acids for use in the disclosure include fatty acids. As used herein, the term "fatty acid" has its ordinary meaning as would be understood by a person of ordinary skill in the art and includes a molecule having a carboxylic group and a hydrocarbon chain. Descriptions of the number of carbon atoms in a fatty acid herein refer to the number of carbon atoms in the hydrocarbon chain of the fatty acid, irrespective of whether the hydrocarbon chain is straight or branched.

As used herein, the term "fatty acid" includes saturated fatty acids, which do not contain any double or triple bonds in the hydrocarbon chain. Saturated fatty acids include, but are not limited to propionic acid (C3) (by way of example, C3 indicates propionic acid has 3 carbon atoms in its hydrocarbon chain; the number of carbon atoms in the hydrocarbon chain of other example fatty acids is denoted in analogous fashion herein), butyric acid (C4), valeric acid (C5), caproic acid (C6), enanthic acid (C7), caprylic acid (C8), pelargonic acid (C9), capric acid (C10), undecylic acid (C11), lauric acid (C12), tridecylic acid (C13), myristic acid (C14), pentadecylic acid (C15), palmitic acid (C16), margaric acid (C17), stearic acid (C18), isostearic acid (C18), nonadecylic acid (C19), arachidic acid (C20), heneicosylic acid (C21), behenic acid (C22), tricosylic acid (C23), lignoceric acid (C24), pentacosylic acid (C25), cerotic acid (C26), heptacosylic acid (C27), montanic acid (C28), nonacocylic acid (C29), melissic acid (C30), henatriacontylic acid (C31), lacceroic acid (C32), psyllic acid (C33), geddic acid (C34), ceroplastic acid (C35) and hexatriacontylic acid (C36).

As used herein, the term "fatty acid" also includes monounsaturated fatty acids, which contain one double or triple bond in the hydrocarbon chain, and polyunsaturated fatty acids, which contain more than one double and/or triple bond in the hydrocarbon chain. Such acids include, but are not limited to the omega 3, omega 6, omega 9 fatty acids, other fatty acids such as myristoleic and palmitoleic acid and conjugated fatty acids. Examples of monounsaturated and polyunsaturated fatty acids include but are not limited to, (a) omega 3 fatty acids, such as hexadecatrienoic acid (C16:3); (by way of example, C16:3 indicates hexadecatrienoic acid has 16 carbon atoms in its hydrocarbon chain and 3 double bonds; the number of carbon atoms and double bonds in the hydrocarbon chain of other example unsaturated fatty acids is denoted in analogous fashion herein), alpha linolenic acid (C18:3) and eicosapentanoic acid (20:5), (b) omega 6 fatty acids, such as linoleic acid (18:2), docosadienoic acid (C22:2), arachidonic acid (C20:4) and tetracosatetraenoic acid (C24:5), (c) omega 9 fatty acids, such as oleic acid (C18:1), eicosenoic acid (C20:1) and nevronic acid (C24:1), and (d) conjugated fatty acids such as rumenic acid (C18:2), eleostatic acid (C18:3), and rumelenic acid (C18:3).

As used herein, the term "fatty acid" also includes branched fatty acids. Examples of branched fatty acids include, but are not limited to, monomethyl branched fatty acids, such as 14-methyl pentadecanoic acid, 6-methyl caprylic acid, 4-methyl-3-pentenoic acid, (pyroterebic acid), 2-methyl-2E-butenoic acid (tiglic acid), 2-methyl-2Z-butenoic acid (angelic acid), multimethyl branched acids, isoprenoid fatty acids (vittatalactone, all-trans-retinoic acid), branched methoxy fatty acids and hydroxy and other fatty acids such as 2-hydroxyoctanoic acid and 4-oxopentanoic acid.

The compositions of the disclosure can comprise from about 0.01 vol.% to about 15 vol.% of the organic acid. In some embodiment, the compositions comprise from about 1 vol% to about 15 vol% of the organic acid. In preferred embodiments, the compositions comprise from about 0.01 vol.% to about 5 vol.% of the organic acid. In other embodiments, the compositions comprise from about 0.01 vol.% to about 3 vol.% of the organic acid. For example, the compositions can comprise about 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, or about 15 vol.% of the organic acid.

According to the disclosure, the compositions also include a therapeutic agent of the formula (IV):

R⁴-N=C=Z (IV)

wherein Z is S or O, and R⁴ is C₁₋₆alkyl, C₂₋₆alkenyl, C₁₋₆alk-aryl, or aryl.

In some embodiments, the compound R⁴ in the compound of formula (IV) will be substituted with one or more substituents. In some embodiments, the substituents on R⁴ are halogen atoms (F, Cl, Br, or I), C₁₋₆alkoxy groups, C₁₋₆alkyl groups, or methylsulfinyl groups..

In some embodiments, Z in the compound of formula (IV) is S (*i.e.*, sulfur), and the compounds of formula (IV) are isothiocyanates of the formula R⁴-N=C=S wherein R⁴ is C₁₋₆alkyl, C₂₋₆alkenyl, C₁₋₆alk-aryl, or aryl.

In embodiments wherein Z is S and R⁴ is C₁₋₆alkyl, the compounds of formula (IV) are isothiocyanates of the formula C₁₋₆alkyl-N=C=S, and include for example C₁alkyl-N=C=S, C₂alkyl-N=C=S, C₃alkyl-N=C=S, C₄alkyl-N=C=S, Csalkyl-N=C=S, C₆alkyl-N=C=S, methyl isothiocyanate, substituted methyl isothiocyanate, ethyl isothiocyanate, substituted ethyl isothiocyanate, propyl isothiocyanate, substituted propyl isothiocyanate, butyl isothiocyanate, t-butyl isothiocyanate, substituted butyl isothiocyanate, pentyl isothiocyanate, substituted pentyl isothiocyanate, hexyl isothiocyanate, substituted hexyl isothiocyanate and the like. In some embodiments, the C₁₋₆alkyl is substituted with one or more methylsulfinyl groups. Thus, in some embodiments, the compound of formula (IV) is a methylsulfinyl-substituted butyl isothiocyanate. In some embodiments, the compound for formula (IV) is sulforaphane (*i*.*e*., 1-isothiocyanato-4-methylsulfinylbutane).

In embodiments wherein Z is S and R⁴ is C₂₋₆alkenyl, the compounds of formula (IV) are isothiocyanates of the formula C₂₋₆alkenyl-N=C=S, and include for example C₂alkenyl-N=C=S, C₃alkenyl-N=C=S, C₄alkenyl-N=C=S, C₅alkenyl-N=C=S, C₆alkenyl-N=C=S, ethenyl isothiocyanate, substituted ethenyl isothiocyanate, propenyl isothiocyanate, substituted propenyl isothiocyanate, butenyl isothiocyanate, substituted butenyl isothiocyanate, pentenyl isothiocyanate, substituted pentenyl isothiocyanate, hexenyl isothiocyanate, substituted hexenyl isothiocyanate and the like. In some embodiments, the C₂₋₆alkenyl group is an allyl group. Thus, in some embodiments, the compound of formula (IV) is allyl isothiocyanate (*i.e*., CH₂=CH-CH₂-N=C=S).

In embodiments wherein Z is S and R⁴ is C₁₋₆alk-aryl, the compounds of formula (IV) are isothiocyanates of the formula aryl-C₁₋₆alk-N=C=S, and include for example aryl-C₁alk-N=C=S, aryl-C₂alk-N=C=S, aryl-C₃alk-N=C=S, aryl-C₄alk-N=C=S, aryl-C₅alk-N=C=S, aryl-C₆alk-N=C=S, benzyl isothiocyanate (*i.e*., phenyl-CH₂-N=C=S), substituted benzyl isothiocyanate, phenethyl isothiocyanate (*i.e*., phenyl-CH₂CH₂-N=C=S, "PEITC"), substituted phenethyl isothiocyanate, 3-phenylpropyl isothiocyanate, substituted 3-phenylpropyl isothiocyanate, and the like.

In some embodiments, the compound of formula (IV) is phenethyl isothiocyanate. In other embodiments, the compound of formula (IV) is a substituted phenethyl isothiocyanate wherein the phenethyl group is substituted with halogen, C₁₋₆alkoxy, or C₁₋₆alkyl.

In some embodiments, the compound of formula (IV) is benzyl isothiocyanate. In other embodiments, the compound of formula (IV) is a substituted benzyl isothiocyanate wherein the benzyl group is substituted with halogen, C₁₋₆alkoxy, or C₁₋₆alkyl.

In embodiments wherein Z is S and R⁴ is aryl, the compounds of formula (IV) are isothiocyanates of the formula aryl-N=C=S, and include for example phenyl isothiocyanate (*i.e*., phenyl-N=C=S), substituted phenyl isothiocynate (*i.e*., substituted phenyl-N=C=S), and the like. In some embodiments, the phenyl group in substituted phenyl-N=C=S is substituted with halogen, C₁₋₆alkoxy, or C₁₋₆alkyl.

In some embodiments, Z in the compound of formula (IV) is O (*i*.*e*., oxygen), and the compounds of formula (IV) are isocyanates of the formula R⁴-N=C=O wherein R⁴ is C₁₋₆alkyl, C₂₋₆alkenyl, C₁₋₆alk-aryl, or aryl.

In embodiments wherein Z is O and R⁴ is C₁₋₆alkyl, the compounds of formula (IV) are isocyanates of the formula C₁₋₆alkyl-N=C=O, and include for example C₁alkyl-N=C=O, C₂alkyl-N=C=O, C₃alkyl-N=C=O, C₄alkyl-N=C=O, C₅alkyl-N=C=O, C₆alkyl-N=C=O, methyl isocyanate, substituted methyl isocyanate, ethyl isocyanate, substituted ethyl isocyanate, propyl isocyanate, substituted propyl isocyanate, butyl isocyanate, substituted butyl isocyanate, pentyl isocyanate, substituted pentyl isocyanate, hexyl isocyanate, substituted hexyl isocyanate and the like. In some embodiments, the compound of formula (IV) is t-butyl isocyanate.

In embodiments wherein Z is O and R⁴ is C₂₋₆alkenyl, the compounds of formula (IV) are isocyanates of the formula C₂₋₆alkenyl-N=C=O, and include for example C₂alkenyl-N=C=O, C₃alkenyl-N=C=O, C₄alkenyl-N=C=O, C₅alkenyl-N=C=O, C₆alkenyl-N=C=O, ethenyl isocyanate, substituted ethenyl isocyanate, propenyl isocyanate, substituted propenyl isocyanate, butenyl isocyanate, substituted butenyl isocyanate, pentenyl isocyanate, substituted pentenyl isocyanate, hexenyl isocyanate, substituted hexenyl isocyanates and the like. In some embodiments, the C₂₋₆alkenyl group is an allyl group. Thus, in some embodiments, the compound of formula (IV) is allyl isocyanate (*i*.*e*., CH₂=CH-CH₂-N=C=O).

In embodiments wherein Z is O and R⁴ is C₁₋₆alk-aryl, the compounds of formula (IV) are isothiocyanates of the formula aryl-C₁₋₆alk-N=C=O, and include for example aryl-C₁alk-N=C=O, aryl-C₂alk-N=C=O, aryl-C₃alk-N=C=O, aryl-C₄alk-N=C=O, aryl-C₅alk-N=C=O, aryl-C₆alk-N=C=O, benzyl isocyanate (*i.e*., phenyl-CH₂-N=C=O), substituted benzyl isocyanate, phenethyl isocyanate (*i.e*., phenyl-CH₂CH₂-N=C=O), substituted phenethyl isocyanate, 3-phenylpropyl isocyanate, substituted 3-phenylpropyl isocyanate, and the like.

In some embodiments, the compound of formula (IV) is phenethyl isocyanate. In other embodiments, the compound of formula (IV) is a substituted phenethyl isocyanate wherein the phenethyl group is substituted with halogen, C₁₋₆alkoxy, or C₁₋₆alkyl.

In other embodiments, the compound of formula (IV) is benzyl isocyanate. In other embodiments, the compound of formula (IV) is a substituted benzyl isocyanate wherein the benzyl group is substituted with halogen, C₁₋₆alkoxy, or C₁₋₆alkyl.

In embodiments wherein Z is O and R⁴ is aryl, the compounds of formula (IV) are isocyanates of the formula aryl-N=C=O, and include for example phenyl isocyanate (*i.e*., phenyl-N=C=O), substituted phenyl isothiocyante (*i.e*., substituted phenyl-N=C=O), and the like. In some embodiments, the phenyl group in substituted phenyl-N=C=O is substituted with halogen, C₁₋₆alkoxy, or C₁₋₆alkyl.

Where the therapeutic agent of the formula (IV) is a liquid at standard temperature and pressure (0°C, 1 bar absolute pressure), the compositions of the disclosure can comprise from about 0.01 vol.% to about 15 vol.% of the therapeutic agent of the formula (IV). In some embodiments, the compositions comprise from about 1 vol% to about 15 vol% of the therapeutic agent of the formula (IV). In preferred embodiments, the compositions comprise from about 0.01 vol.% to about 5 vol.% of the therapeutic agent of the formula (IV). In other embodiments, the compositions comprise from about 0.01 vol.% to about 3 vol.% of the therapeutic agent of the formula (IV). For example, the compositions can comprise about 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, or about 15 vol.% of the therapeutic agent of the formula (IV).

Where the therapeutic agent of the formula (IV) is a solid at standard temperature and pressure (0°C, 1 bar absolute pressure), the compositions of the disclosure can comprise from about 0.01 wt.% to about 15 wt.% of the therapeutic agent of the formula (IV). In some embodiments, the compositions comprise from about 1 wt% to about 15 wt% of the therapeutic agent of the formula (IV). In preferred embodiments, the compositions comprise from about 0.01 wt.% to about 5 wt.% of the therapeutic agent of the formula (IV). In other embodiments, the compositions comprise from about 0.01 wt.% to about 3 wt.% of the therapeutic agent of the formula (IV). For example, the compositions can comprise about 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, or about 15 wt.% of the therapeutic agent of the formula (IV).

In some embodiments, the compositions of the present disclosure also include a second therapeutic agent that is an anticancer agent. Exemplary anticancer agents that may be present in the compositions of the present disclosure as a second therapeutic agent include 5-fluorouracil, abiraterone acetate, acetylcholine, ado-trastuzumab emtansine, afatinib, aldesleukin, alectinib, alemtuzumab, alitretinoin, aminolevulinic acid, anastrozole, anastrozole, aprepitant, arsenic trioxide, asparaginase erwinia chrysanthemi, atezolizumab, axitinib, azacitidine, belinostat, bendamustine, bevacizumab, bexarotene, bicalutamide, bleomycin, blinatumomab, bortezomib, bosutinib, brentuximab vedotin, busulfan, cabazitaxel, cabozantinib, capecitabine, carboplatin, carfilzomib, carmustine, ceritinib, cetuximab, chlorambucil, cisplatin, clofarabine, cobimetinib, crizotinib, cyclophosphamide, cytarabine, dabrafenib, dacarbazine, dacarbazine, dactinomycin, daratumumab, dasatinib, daunorubicin, decitabine, defibrotide sodium, degarelix, denileukin diftitox, denosumab, dexamethasone, dexrazoxane, dihydrotestosterone (DHT), dinutuximab, docetaxel, doxorubicin, elotuzumab, eltrombopag, enzalutamide, epirubicin, eribulin mesylate, erlotinib, etoposide, everolimus, exemestane, exemestane, filgrastim, fludarabine phosphate, flutamide, fulvestrant, fulvestrant, gefitinib, gemcitabine, gemtuzumab, gemtuzumab ozogamicin, glucarpidase, goserelin acetate, hydroxyurea, ibritumomab tiuxetan, ibrutinib, idarubicin, idelalisib, ifosfamide, imatinib, imiquimod, interferon alfa-2b, ipilimumab, irinotecan, ixabepilone, ixazomib, lanreotide, lapatinib, lenalidomide, lenvatinib, letrozole, leucovorin, leuprolide, lomustine, mechlorethamine, megestrol acetate, melphalan, mercaptopurine, mesna, methotrexate, mitomycin C, mitoxantrone, necitumumab, nelarabine, netupitant, nilotinib, nilutamide, nivolumab, obinutuzumab, ofatumumab, olaparib, omacetaxine mepesuccinate, osimertinib, oxaliplatin, ozogamicin, paclitaxel, palbociclib, palifermin, pamidronate, panitumumab, panobinostat, pazopanib, pegaspargase, peginterferon alfa-2b, pembrolizumab, pemetrexed, pertuzumab, plerixafor, pomalidomide, ponatinib, pralatrexate, prednisone, procarbazine, propranolol, radium 223 dichloride, raloxifene, ramucirumab, rasburicase, regorafenib, rituximab, rolapitant, romidepsin, romiplostim, ruxolitinib, siltuximab, sipuleucel-t, sonidegib, sorafenib, sunitinib, talimogene laherparepvec, tamoxifen, temozolomide, temsirolimus, thalidomide, thioguanine, thiotepa, tipiracil, topotecan, toremifene, toremifene, tositumomab, trabectedin, trametinib, trastuzumab, tretinoin, trifluridine, uridine triacetate, vandetanib, vemurafenib, venetoclax, vinblastine, vincristine, vinorelbine, vismodegib, vorinostat, ziv-aflibercept, zoledronic acid, and pharmaceutically acceptable salts thereof.

Another anticancer agent that may be included in the compositions of the present disclosure is SM88, five components (sirolimus, melanin, melanotan, phenytoin, and tyrosine isomers) that when administered together, has demonstrated anti-cancer activity with little to no toxicity in a preliminary study of 30 patients [J Clin Oncol 31, 2013 (suppl; abstr e22095) and Hoffman et al. GynOncol, 130(1), 2013, e43]. Thus, in some embodiments the compositions of the present disclosure comprise both a compound of formula (IV), and one of more components of SM88 as a second therapeutic agent.

In some embodiments, the compositions of the present disclosure comprise benzyl isothiocyanate or phenethyl isothiocyanate as the compound of formula (IV), and one or more components of SM88 as a second therapeutic agent.

Where the second therapeutic agent is a liquid at standard temperature and pressure (0°C, 1 bar absolute pressure), the compositions of the disclosure can comprise from about 0.01 vol.% to about 15 vol.% of the second therapeutic agent. In some embodiments, the compositions comprise from about 1 vol% to about 15 vol% of the second therapeutic agent. In preferred embodiments, the compositions comprise from about 0.01 vol.% to about 5 vol.% of the second therapeutic agent. In other embodiments, the compositions comprise from about 0.01 vol.% to about 3 vol.% of the second therapeutic agent. For example, the compositions can comprise about 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, or about 15 vol.% of the second therapeutic agent.

Where the second therapeutic agent is a solid at standard temperature and pressure (0°C, 1 bar absolute pressure), the compositions of the disclosure can comprise from about 0.01 wt.% to about 15 wt.% of the second therapeutic agent. In some embodiments, the compositions comprise from about 1 wt% to about 15 wt% of the second therapeutic agent. In preferred embodiments, the compositions comprise from about 0.01 wt.% to about 5 wt.% of the second therapeutic agent. In other embodiments, the compositions comprise from about 0.01 wt.% to about 3 wt.% of the second therapeutic agent. For example, the compositions can comprise about 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, or about 15 wt.% of the second therapeutic agent.

Compositions of the disclosure can be anhydrous. As used herein, "anhydrous" refers to compositions comprising less than 1 vol.% of water, preferably less than 0.05 vol.% or less than 0.025 vol.% of water. Methods of determining water content are known in the art.

Compositions of the disclosure can include water. In some embodiments, the compositions can comprise up to 99 vol.% of water. In still other aspects, the compositions can comprise 5, 10, 20, 30, 40, 50, 60, 70, 80, 90, 95, or 99 vol.% of water. In other embodiments, the compositions can comprise 90, 91, 92, 93, 94, 95, 96, 97, 98, or 99 vol.% of water.

Compositions of the disclosure that include water can optionally contain one or more physiologically acceptable salts. While not being bound by any particular theory, it is believed that controlling the amount of salt that is present allows one to control the depth to which the present composition penetrate skin, with the concentration of salt having a generally inverse relationship to the penetration depth. Salts for use in the compositions include, but are not limited to, sodium chloride, potassium chloride, and mixtures thereof. A preferred form of sodium chloride is bacteriostatic sodium chloride solution.

Compositions of the invention may be designed to be administered to the skin or mucosal tissue of a patient in need of treatment. Compositions of the invention may be formulated as gels, transdermal patches, lotions, creams, sprays, mists, emulsions, or dispersions. Appropriate excipients for formulating a gel, transdermal patch, lotion, cream, spray, or mist are readily apparent to a person of skill in the art and include, but are not limited to, stabilizers, emulsifiers, thickeners, antimicrobials, humectants, propellants, spreading agents, polymers, and adhesives, such as pressure sensitive adhesives. In particular, excipients that may be used to form a transdermal gel include, but are not limited to, alcohols, glycols, glycerin, butylated hydroxytoluene (BHT), and water.

Also within the scope of the disclosure are compositions for use in methods comprising administering any of the described compositions to the skin of a mammal for a time and under conditions effective to achieve passage of at least a portion of the composition through the skin. Skin permeation can be measured using techniques known in the art.

The compositions of the disclosure can be used to administer a therapeutic agent (e.g., a compound of formula (IV)) to a mammal. For example, in preferred embodiments, these methods comprise applying any of the described compositions to the skin of a mammal for a time sufficient to achieve permeation of at least a portion of the therapeutic agent through the skin. Therapeutic agent skin permeation can be measured using techniques known in the art.

The disclosure further encompasses compositions for use in methods of treating cancer in a mammal in need thereof comprising applying a composition of the present disclosure to the skin or a mucous membrane of said mammal for a time sufficient to achieve permeation of a effective amount of the therapeutic agent of formula (IV) through the skin or the mucous membrane. In some embodiments, the mammal is a human being. In some embodiments, the cancer is non-small cell lung cancer, ovarian cancer, breast cancer, cervical cancer, pancreatic cancer, stomach cancer, brain cancer, liver cancer, testicular cancer, leukemia, lymphoma, appendix cancer, biliary cancer, choleangiocarcinoma, colon cancer, colorectal cancer, germ cell tumor, glioma, Hodgkin's lymphoma, lung cancer, neuroblastoma, prostate cancer, renal cancer, sarcoma, thyroid cancer, tongue cancer, tonsil squamous cell carcinoma, or urothelial cancer. In some embodiments, the cancer is leukemia. In other embodiments, the cancer is lung cancer.

In some embodiments, the compositions of the present disclosure are administered as a component of a combination chemotherapy, whereby a composition of the present disclosure is administered in conjunction with another chemotherapeutic regimen. The other chemotherapeutic regimen may comprise administering to the mammal conventional anticancer agents by any suitable route of administration. As used herein, the term "in conjunction with" means that the compositions of the present disclosure and the other chemotherapeutic regimen are administered such that at some point in time, a compound of formula (IV) and a conventional anticancer agent are both present within the mammal's bloodstream. In some embodiments, the other chemotherapeutic regimen comprises administering to the mammal in need of chemotherapy an effective amount of 5-fluorouracil, abiraterone acetate, acetylcholine, ado-trastuzumab emtansine, afatinib, aldesleukin, alectinib, alemtuzumab, alitretinoin, aminolevulinic acid, anastrozole, anastrozole, aprepitant, arsenic trioxide, asparaginase erwinia chrysanthemi, atezolizumab, axitinib, azacitidine, belinostat, bendamustine, bevacizumab, bexarotene, bicalutamide, bleomycin, blinatumomab, bortezomib, bosutinib, brentuximab vedotin, busulfan, cabazitaxel, cabozantinib, capecitabine, carboplatin, carfilzomib, carmustine, ceritinib, cetuximab, chlorambucil, cisplatin, clofarabine, cobimetinib, crizotinib, cyclophosphamide, cytarabine, dabrafenib, dacarbazine, dacarbazine, dactinomycin, daratumumab, dasatinib, daunorubicin, decitabine, defibrotide sodium, degarelix, denileukin diftitox, denosumab, dexamethasone, dexrazoxane, dihydrotestosterone (DHT), dinutuximab, docetaxel, doxorubicin, elotuzumab, eltrombopag, enzalutamide, epirubicin, eribulin mesylate, erlotinib, etoposide, everolimus, exemestane, exemestane, filgrastim, fludarabine phosphate, flutamide, fulvestrant, fulvestrant, gefitinib, gemcitabine, gemtuzumab, gemtuzumab ozogamicin, glucarpidase, goserelin acetate, hydroxyurea, ibritumomab tiuxetan, ibrutinib, idarubicin, idelalisib, ifosfamide, imatinib, imiquimod, interferon alfa-2b, ipilimumab, irinotecan, ixabepilone, ixazomib, lanreotide, lapatinib, lenalidomide, lenvatinib, letrozole, leucovorin, leuprolide, lomustine, mechlorethamine, megestrol acetate, melphalan, mercaptopurine, mesna, methotrexate, mitomycin C, mitoxantrone, necitumumab, nelarabine, netupitant, nilotinib, nilutamide, nivolumab, obinutuzumab, ofatumumab, olaparib, omacetaxine mepesuccinate, osimertinib, oxaliplatin, ozogamicin, paclitaxel, palbociclib, palifermin, pamidronate, panitumumab, panobinostat, pazopanib, pegaspargase, peginterferon alfa-2b, pembrolizumab, pemetrexed, pertuzumab, plerixafor, pomalidomide, ponatinib, pralatrexate, prednisone, procarbazine, propranolol, radium 223 dichloride, raloxifene, ramucirumab, rasburicase, regorafenib, rituximab, rolapitant, romidepsin, romiplostim, ruxolitinib, siltuximab, sipuleucel-t, SM88, sonidegib, sorafenib, sunitinib, talimogene laherparepvec, tamoxifen, temozolomide, temsirolimus, thalidomide, thioguanine, thiotepa, tipiracil, topotecan, toremifene, toremifene, tositumomab, trabectedin, trametinib, trastuzumab, tretinoin, trifluridine, uridine triacetate, vandetanib, vemurafenib, venetoclax, vinblastine, vincristine, vinorelbine, vismodegib, vorinostat, ziv-aflibercept, zoledronic acid, or pharmaceutically acceptable salts thereof.

In some embodiments, the other chemotherapeutic regimen comprises administering to the mammal in need of chemotherapy an effective amount of SM88, cisplatin, adriamycin, etoposide, paclitaxel, metformin, vorinostat or docetaxel.

The disclosure further encompasses compositions for use in methods of preventing cancer in a mammal in need thereof comprising applying a composition of the present disclosure to the skin or a mucous membrane of said mammal for a time sufficient to achieve permeation of at least a portion of the therapeutic agent of formula (IV) through the skin or the mucous membrane. In some embodiments, the mammal is a human being.

The compositions described herein can be applied to any convenient skin surface. Skin surfaces of interest include, but are not limited to: arms, leg, torso, head, neck, etc. The surface area that is covered by the transdermal formulation following application is generally sufficient to provide for the desired amount of agent administration, and in certain embodiments ranges from about 1 cm² to about 200 cm².

The compositions described herein can be applied a single time or a plurality of times over a given time period, e.g., the course of the disease condition being treated, where the dosing schedule when a plurality of patches are administered over a given time period may be daily, weekly, biweekly, monthly, etc.

The compositions of the disclosure will, in some embodiments, include, in addition to the above-discussed components, one or more additional components. Additional components include, but are not limited to, a transdermal absorption enhancer, a preservative (e.g., paraben), an antioxidant, a stabilizing agent, a filling agent that contains a hydrophilic polymer; a cross-linking agents; and a plasticizing agent.

The following example is provided to illustrate the compositions, processes, and properties of the present disclosure. The example is merely illustrative and not intended to limit the disclosure to the materials, conditions, or process parameters set forth therein.

### EXAMPLES

### Example 1. Aqueous composition for transdermal administration of a therapeutic agent of formula (IV)

Nonaethylene glycol monododecyl ether (3 mL), 1-methyl-2-pyrrolidinone (0.3 mL), ethanol (4 mL), oleic acid (1 mL), and water (50 mL) are combined to form an admixture. An effective amount of a therapeutic agent of formula (IV) is combined with the admixture to form a transdermal composition. The transdermal composition is applied to the skin of a patient in an amount and for a time sufficient for the therapeutic agent to permeate through the skin and into the patient's bloodstream to achieve a therapeutic effect.

### Example 2. Anhydrous composition for transdermal administration of a therapeutic agent of formula (IV)

Nonaethylene glycol monododecyl ether (3 mL), 1-methyl-2-pyrrolidinone (0.3 mL), ethanol (4 mL), and linoleic acid (1 mL) are combined to form an admixture. An effective amount of a therapeutic agent of formula (IV) is combined with the admixture to form a transdermal composition.

### Example 3. Transdermal administration of a therapeutic agent of formula (IV) using an anhydrous composition.

The transdermal composition of Example 2 is applied to the skin of a patient in an amount and for a time sufficient for the therapeutic agent of formula (IV) to permeate through the skin and into the patient's bloodstream to achieve a therapeutic effect.

### Example 4. Aqueous, sensitive tissue transdermal composition

The composition of Example 2 (1 mL) is mixed with 99 mL of water. The resulting aqueous composition can be applied to a sensitive tissue, for example a mucous membrane, for a time sufficient for the therapeutic agent to permeate through the sensitive tissue and into the patient's bloodstream to achieve a therapeutic effect.

### Example 5. Aqueous, normal skin transdermal composition

The composition of Example 2 (1 mL) is mixed with water 49 mL of water. The resulting aqueous composition can be applied to normal skin for a time sufficient for the therapeutic agent to permeate through the skin and into the patient's bloodstream to achieve a therapeutic effect.

### Example 6. Aqueous, phenethyl isothiocyanate transdermal composition

Nonaethylene glycol monododecyl ether (3 mL), 1-methyl-2-pyrrolidinone (0.3 mL), ethanol (4 mL), and linoleic acid (1 mL) are combined. Phenethyl isothiocyanate (1 mL) (e.g., Sigma Aldrich catalog number 253731) is then added to form an admixture. The admixture (1 mL) is then combined with 24 mL of water. The resulting aqueous composition can be applied to skin or tissue for a time sufficient for the phenethyl isothiocyanate to permeate through the skin or tissue and into the patient's bloodstream to achieve a therapeutic effect.

### Example 7. Aqueous, benzyl isothiocyanate transdermal composition

Nonaethylene glycol monododecyl ether (3 mL), 1-methyl-2-pyrrolidinone (0.3 mL), ethanol (4 mL), and linoleic acid (1 mL) are combined. Benzyl isothiocyanate (1 mL) (e.g., Sigma Aldrich catalog number 252492) is then added to form an admixture. The admixture (1 mL) is then combined with 24 mL of water. The resulting aqueous composition can be applied to skin or tissue for a time sufficient for the benzyl isothiocyanate to permeate through the skin or tissue and into the patient's bloodstream to achieve a therapeutic effect.

### Example 8. Aqueous, phenethyl isothiocyanate transdermal composition

Nonaethylene glycol monododecyl ether (3 mL), 1-methyl-2-pyrrolidinone (0.3 mL), ethanol (4 mL), and linoleic acid (1 mL) are combined. Phenethyl isothiocyanate (1 mL) (e.g., Sigma Aldrich catalog number 253731) is then added to form an admixture. The admixture (1 mL) is then combined with 32.3 mL of water. The resulting aqueous composition can be applied to skin or tissue for a time sufficient for the phenethyl isothiocyanate to permeate through the skin or tissue and into the patient's bloodstream to achieve a therapeutic effect.

### Example 9. Aqueous, benzyl isothiocyanate transdermal composition

Nonaethylene glycol monododecyl ether (3 mL), 1-methyl-2-pyrrolidinone (0.3 mL), ethanol (4 mL), and linoleic acid (1 mL) are combined. Benzyl isothiocyanate (1 mL) (e.g., Sigma Aldrich catalog number 252492) is then added to form an admixture. The admixture (1 mL) is then combined with 32.3 mL of water. The resulting aqueous composition can be applied to skin or tissue for a time sufficient for the benzyl isothiocyanate to permeate through the skin or tissue and into the patient's bloodstream to achieve a therapeutic effect.

## Claims

1. A composition comprising a therapeutic agent of the formula (IV):
R⁴-N=C=Z (IV)
wherein Z is S or O, and R⁴ is C₁₋₆alkyl, C₂₋₆alkenyl, C₁₋₆alk-aryl, or aryl, and:
- a compound of the formula (I)
R-(OCH₂CH₂)_{y}-OH (I)
wherein R is C₁₋₂₀alkyl or C₂₋₂₀alkenyl; and y is 1 to 25;
- an amide of the formula (III)
R²-N(R¹)-C(O)-R³ (III)
wherein each R¹ is independently H or C₁₋₃alkyl; and R² and R³ together with the atoms to which they are attached, form a lactam having 3 to 10 carbon atoms;
- a C₂₋₁₀ alkyl alcohol;
- an organic acid that is a fatty acid, having 1 to 25 carbon atoms.

2. The composition of claim 1, wherein R is C₁₋₂₀alkyl; or wherein the compound of formula (I) is cetomacrogol 1000; octadecan-1-ol, ethoxylated; polyoxyethylene(12)tridecyl ether; polyoxyethylene(10)tridecyl ether; fatty alcohol polyoxyethylene ether, polyoxyethylene branched nonylcyclohexyl ether, nonaethylene glycol monododecyl ether, 23-{[4-(2,4,4-trimethyl-2-pentanyl)cyclohexyl]oxy}-3,6,9,12,15,18,21-heptaoxatricosan-1-ol, or a combination thereof; or wherein the compound of formula (I) is nonaethylene glycol monododecyl ether.

3. The composition of claim 1, wherein R is C₂₋₂₀alkenyl; or wherein the compound of formula (I) is polyoxyl(10)oleyl ether, polyethylene glycol tert-octylphenyl ether, or a combination thereof.

4. The composition of claim 1, wherein y is 5 to 15; or wherein y is 8 to 10; or wherein y is 9.

5. The composition of claim 1, wherein R¹ is methyl, ethyl, or propyl.

6. The composition of claim 1, wherein the lactam is a pyrrolidone; preferably wherein the pyrrolidone is 2-pyrrolidone, 1-methyl-2-pyrrolidone, 5-methyl-2-pyrrolidone, or 1-ethyl-2-pyrrolidone; most preferably wherein the pyrrolidone is 1-methyl-2-pyrrolidone.

7. The composition of claim 1, wherein the C₂₋₁₀alkyl alcohol is glycerol, propylene glycol, ethanol, isopropanol, 1-propanol, butanol, t-butanol, pentanol, 1-octanol, or a combination thereof; most preferably wherein the C₂₋₁₀alkyl alcohol is ethanol.

8. The composition of claim 1, wherein the fatty acid is a saturated fatty acid, an unsaturated fatty acid, or a polyunsaturated fatty acid; or wherein the fatty acid is butyric acid, valeric acid, caproic acid, enanthic acid, caprylic acid, pelargonic acid, capric acid, undecylic acid, lauric acid, tridecylic acid, myristic acid, pentadecylic acid, palmitic acid, margaric acid, stearic acid, isostearic acid, nonadecylic acid, arachidic acid, heneicosylic acid, behenic acid, tricosylic acid, lignoceric acid, pentacosylic acid, cerotic acid, heptacosylic acid, montanic acid, nonacocylic acid, melissic acid, henatriacontylic acid, lacceroic acid, psyllic acid, geddic acid, ceroplastic acid, or hexatriacontylic acid; or wherein the fatty acid is hexadecatrienoic acid, alpha linolenic acid, eicosapentanoic acid, linoleic acid, docosadienoic acid, arachidonic acid, tetracosatetraenoic acid, oleic acid, eicosenoic acid, nevronic acid, rumenic acid, eleostatic acid, or rumelenic acid; or wherein the fatty acid is 14-methyl pentadecanoic acid, 6-methyl caprylic acid, 4-methyl-3-pentenoic acid, (pyroterebic acid), 2-methyl-2E-butenoic acid (tiglic acid), 2-methyl-2Z-butenoic acid (angelic acid), vittatalactone (all-trans-retinoic acid), 2-hydroxyoctanoic acid, or 4-oxopentanoic acid.

9. The composition of claim 1, wherein Z in the compound of formula (IV) is S; or wherein R⁴ in the compound of formula (IV) is C₁₋₆alkyl; or wherein the compound of formula (IV) is methyl isothiocyanate, substituted methyl isothiocyanate, ethyl isothiocyanate, substituted ethyl isothiocyanate, propyl isothiocyanate, substituted propyl isothiocyanate, butyl isothiocyanate, t-butyl isothiocyanate, substituted butyl isothiocyanate, pentyl isothiocyanate, substituted pentyl isothiocyanate, hexyl isothiocyanates, or substituted hexyl isothiocyanate; or wherein the propyl isothiocyanate is isopropyl isothiocyanate; or wherein the butyl isothiocyanate is t-butyl isothiocyanate; or wherein the substituted butyl isothiocyanate is 1-isothiocyanato-4-methylsulfinylbutane.

10. The composition of claim 9, wherein R⁴ in the compound of formula (IV) is C₂₋₆alkenyl; or wherein the compound of formula (IV) is ethenyl isothiocyanate, substituted ethenyl isothiocyanate, propenyl isothiocyanate, substituted propenyl isothiocyanate, butenyl isothiocyanate, substituted butenyl isothiocyanate, pentenyl isothiocyanate, substituted pentenyl isothiocyanate, hexenyl isothiocyanate, substituted hexenyl isothiocyanate; or wherein the propenyl isothiocyanate is allyl isothiocyanate.

11. The composition of claim 9, wherein R⁴ in the compound of formula (IV) is -C₁₋₆alk-aryl; or wherein the compound of formula (IV) is benzyl isothiocyanate, substituted benzyl isothiocyanate, phenethyl isothiocyanate, substituted phenethyl isothiocyanate, 3-phenylpropyl isothiocyanate, or substituted 3-phenylpropyl isothiocyanate.

12. The composition of claim 9, wherein R⁴ in the compound of formula (IV) is aryl; or wherein the compound of formula (IV) is phenyl isothiocyanate or substituted phenyl isothiocyanate; or wherein the substituted phenyl isothiocyanate is substituted with halogen, C₁₋₆alkoxy, or C₁₋₆alkyl.

13. The composition of claim 1, wherein Z in the compound of formula (IV) is O; or wherein R⁴ in the compound of formula (IV) is C₁₋₆ alkyl; or wherein the compound of formula IV is methyl isocyanate, substituted methyl isocyanate, ethyl isocyanate, substituted ethyl isocyanate, propyl isocyanate, substituted propyl isocyanate, butyl isocyanate, substituted butyl isocyanate, pentyl isocyanate, substituted pentyl isocyanate, hexyl isocyanate, or substituted hexyl isocyanate; or wherein the propyl isocyanate is isopropyl isocyanate; or wherein the butyl isocyanate is t-butyl isocyanate; or wherein R⁴ in the compound of formula (IV) is C₂₋₆alkenyl; or wherein the compound of formula (IV) is ethenyl isocyanate, substituted ethenyl isocyanate, propenyl isocyanate, substituted propenyl isocyanate, butenyl isocyanate, substituted butenyl isocyanate, pentenyl isocyanate, substituted pentenyl isocyanate, hexenyl isocyanate, or substituted hexenyl isocyanate; or wherein the propenyl isocyanate is allyl isocyanate; or wherein R⁴ in the compound of formula (IV) is C₁₋₆alk-aryl; or wherein the compound of formula (IV) is benzyl isocyanate, substituted benzyl isocyanate, phenethyl isocyanate, substituted phenethyl isocyanate, 3-phenylpropyl isocyanate, or substituted 3-phenylpropyl isocyanate; or wherein R⁴ in the compound of formula (IV) is aryl; or wherein the compound of formula (IV) is phenyl isocyanate or substituted phenyl isocyanate; or wherein the substituted phenyl isocyanate is substituted with halogen, C₁₋₆alkoxy, or C₁₋₆alkyl.

14. The composition of claim 1, wherein the composition further comprises a second therapeutic agent that is an anticancer drug; or wherein the composition further comprises a second therapeutic agent that is SM88; or wherein the composition further comprises a second therapeutic agent that is one or more components of SM88.

15. The composition of claim 1, wherein the ratio of the compound of formula (I) to the amide of formula (III) is about 10:1.

16. A composition of claim 1 comprising
nonaethylene glycol monododecyl ether;
1-methyl-2-pyrrolidone;
ethanol;
linoleic acid; and
a therapeutic agent that is allyl isothiocyanate, 1-isothiocyanato-4-methylsulfinylbutane, benzyl isothiocyanate, substituted benzyl isothiocyanate, phenethyl isothiocyanate, substituted phenethyl isothiocyanate, benzyl isocyanate, substituted benzyl isocyanate, phenethyl isocyanate, or substituted phenethyl isocyanate;
wherein the composition allows for the transdermal delivery of the therapeutic agent.

17. The composition of claim 16, wherein the composition further comprises a second therapeutic agent that is an anticancer agent; or further comprises a second therapeutic agent that is SM88; or further comprises a second therapeutic agent that is one or more components of SM88.

18. The composition of claim 16, wherein the ratio (v/v) of the nonaethylene glycol monododecyl ether to the 1-methyl-2-pyrrolidone is from 9:1 to 11:1; or wherein the ratio (v/v) of the nonaethylene glycol monododecyl ether to the 1-methyl-2-pyrrolidone is about 10:1.

19. The composition of any one of claims 1 to 18 for use in the treatment or prevention of cancer in a mammal, wherein the composition is applied to the skin or mucus membrane of said mammal for a time sufficient to achieve permeation of an effective amount of the therapeutic agent of formula (IV) through the skin or mucus membrane.

## Patentansprüche

1. Zusammensetzung, umfassend ein Therapeutikum der Formel (IV) :
R⁴-N=C=Z (IV)
wobei Z S oder O ist und R⁴ C₁₋₆-Alkyl, C₂₋₆-Alkenyl, C₁₋₆-Alkaryl, oder Aryl, ist, und:
- eine Verbindung der Formel (I)
R-(OCH₂CH₂)_{y}-OH (I)
wobei R C₁₋₂₀-Alkyl oder C₂₋₂₀-Alkenyl ist; und y 1 bis 25 ist;
- ein Amid der Formel (III)
R²-N(R¹)-C(O)-R³ (III)
worin jedes R¹ unabhängig H oder C₁₋₃-Alkyl ist; und R² und R³ zusammen mit den Atomen, an die sie gebunden sind, ein Lactam mit 3 bis 10 Kohlenstoffatomen bilden;
- ein C₂₋₁₀-Alkylalkohol;
- eine organische Säure, die eine Fettsäure ist, die 1 bis 25 Kohlenstoffatome hat.

2. Zusammensetzung nach Anspruch 1, wobei R C₁₋₂₀-Alkyl ist; oder wobei die Verbindung der Formel (I) Cetomacrogol 1000; Octadecan-1-ol, ethoxyliert; Polyoxyethylen(12)tridecylether; Polyoxyethylen(10)tridecylether; Fettalkoholpolyoxyethylenether, verzweigter Polyoxyethylennonylcyclohexylether, Nonaethylenglycolmonododecylether, 23-{[4-(2,4,4-Trimethyl-2-pentanyl)cyclohexyl]oxy}-3, 6, 9, 12, 15, 18, 21-Heptaoxatricosan-1-ol, oder eine Kombination davon, ist; oder wobei die Verbindung der Formel (I) Nonaethylenglycolmonododecylether ist.

3. Zusammensetzung nach Anspruch 1, wobei R C₂₋₂₀-Alkenyl ist; oder wobei die Verbindung der Formel (I) Polyoxyl(10)oleylether, Polyethylenglycol-tert-octylphenylether, oder eine Kombination davon, ist.

4. Zusammensetzung nach Anspruch 1, wobei y 5 bis 15 ist; oder wobei y 8 bis 10 ist; oder wobei y 9 ist.

5. Zusammensetzung nach Anspruch 1, wobei R¹ Methyl, Ethyl oder Propyl ist.

6. Zusammensetzung nach Anspruch 1, wobei das Lactam ein Pyrrolidon ist; wobei das Pyrrolidon vorzugsweise 2-Pyrrolidon, 1-Methyl-2-pyrrolidon, 5-Methyl-2-pyrrolidon, oder 1-Ethyl-2-pyrrolidon ist; wobei das Pyrrolidon am bevorzugtesten 1-Methyl-2-pyrrolidon ist.

7. Zusammensetzung nach Anspruch 1, wobei der C₂₋₁₀-Alkylalkohol Glycerin, Propylenglycol, Ethanol, Isopropanol, 1-Propanol, Butanol, t-Butanol, Pentanol, 1-Octanol, oder eine Kombination davon, ist; wobei der C₂₋₁₀-Alkylalkohol am bevorzugtesten Ethanol ist.

8. Zusammensetzung nach Anspruch 1, wobei die Fettsäure eine gesättigte Fettsäure, eine ungesättigte Fettsäure, oder eine mehrfach ungesättigte Fettsäure ist; oder wobei die Fettsäure Buttersäure, Valeriansäure, Capronsäure, Enanthinsäure, Caprylsäure, Pelargonsäure, Caprinsäure, Undecylsäure, Laurinsäure, Tridecylsäure, Myristinsäure, Pentadecylsäure, Palmitinsäure, Margarinsäure, Stearinsäure, Isostearinsäure, Nonadecylsäure, Arachinsäure, Heneicosylsäure, Behensäure, Tricosylsäure, Lignocerinsäure, Pentacosylsäure, Cerosäure, Heptacosylsäure, Montansäure, Nonacocylsäure, Melisssäure, Henatriacontylsäure, Lacceroinsäure, Psyllinsäure, Geddinsäure, Ceroplastische Säure, oder Hexatriacontylsäure ist; oder wobei die Fettsäure Hexadecatriensäure, Alpha-Linolensäure, Eicosapentansäure, Linolsäure, Docosadiensäure, Arachidonsäure, Tetracosatetraensäure, Ölsäure, Eicosensäure, Nevronsäure, Pansensäure, Eleostatsäure oder Rumelensäure ist; oder wobei die Fettsäure 14-Methylpentadecansäure, 6-Methylcaprylsäure, 4-Methyl-3-pentensäure (Pyroterebinsäure), 2-Methyl-2E-butensäure (Tiglinsäure), 2-Methyl-2Z-Butensäure (Engelsäure), Vittatalacton (all-trans-Retinsäure), 2-Hydroxyoctansäure, oder 4-Oxopentansäure ist.

9. Zusammensetzung nach Anspruch 1, wobei Z in der Verbindung der Formel (IV) S ist; oder wobei R⁴ in der Verbindung der Formel (IV) C₁₋₆-Alkyl ist; oder wobei die Verbindung der Formel (IV) Methylisothiocyanat, substituiertes Methylisothiocyanat, Ethylisothiocyanat, substituiertes Ethylisothiocyanat, Propylisothiocyanat, substituiertes Propylisothiocyanat, Butylisothiocyanat, t-Butylisothiocyanat, substituiertes Butylisothiocyanat, Pentylisothiocyanat, substituiertes Pentylisothiocyanat, Hexylisothiocyanate oder substituiertes Hexylisothiocyanat ist; oder wobei das Propylisothiocyanat Isopropylisothiocyanat ist; oder wobei das Butylisothiocyanat t-Butylisothiocyanat ist; oder wobei das substituierte Butylisothiocyanat 1-Isothiocyanato-4-methylsulfinylbutan ist.

10. Zusammensetzung nach Anspruch 9, wobei R⁴ in der Verbindung der Formel (IV) C₂₋₆-Alkenyl ist; oder wobei die Verbindung der Formel (IV) Ethenylisothiocyanat, substituiertes Ethenylisothiocyanat, Propenylisothiocyanat, substituiertes Propenylisothiocyanat, Butenylisothiocyanat, substituiertes Butenylisothiocyanat, Pentenylisothiocyanat, substituiertes Pentenylisothiocyanat, Hexenylisothiocyanat, substituiertes Hexenylisothiocyanat, ist; oder wobei das Propenylisothiocyanat Allylisothiocyanat ist.

11. Zusammensetzung nach Anspruch 9, wobei R⁴ in der Verbindung der Formel (IV) -C₁₋₆-Alkaryl ist; oder wobei die Verbindung der Formel (IV) Benzylisothiocyanat, substituiertes Benzylisothiocyanat, Phenethylisothiocyanat, substituiertes Phenethylisothiocyanat, 3-Phenylpropylisothiocyanat oder substituiertes 3-Phenylpropylisothiocyanat ist.

12. Zusammensetzung nach Anspruch 9, wobei R⁴ in der Verbindung der Formel (IV) Aryl ist; oder wobei die Verbindung der Formel (IV) Phenylisothiocyanat oder substituiertes Phenylisothiocyanat ist; oder wobei das substituierte Phenylisothiocyanat mit Halogen, C₁₋₆-Alkoxy oder C₁₋₆-Alkyl substituiert ist.

13. Zusammensetzung nach Anspruch 1, wobei Z in der Verbindung der Formel (IV) O ist; oder wobei R⁴ in der Verbindung der Formel (IV) C₁₋₆-Alkyl ist; oder wobei die Verbindung der Formel IV Methylisocyanat, substituiertes Methylisocyanat, Ethylisocyanat, substituiertes Ethylisocyanat, Propylisocyanat, substituiertes Propylisocyanat, Butylisocyanat, substituiertes Butylisocyanat, Pentylisocyanat, substituiertes Pentylisocyanat, Hexylisocyanat oder substituiertes Hexylisocyanat ist; oder wobei das Propylisocyanat Isopropylisocyanat ist; oder wobei das Butylisocyanat t-Butylisocyanat ist; oder wobei R⁴ in der Verbindung der Formel (IV) C₂₋₆-Alkenyl ist; oder wobei die Verbindung der Formel (IV) Ethenylisocyanat, substituiertes Ethenylisocyanat, Propenylisocyanat, substituiertes Propenylisocyanat, Butenylisocyanat, substituiertes Butenylisocyanat, Pentenylisocyanat, substituiertes Pentenylisocyanat, Hexenylisocyanat oder substituiertes Hexenylisocyanat ist; oder wobei das Propenylisocyanat Allylisocyanat ist; oder wobei R⁴ in der Verbindung der Formel (IV) C₁₋₆-Alk-Aryl ist; oder wobei die Verbindung der Formel (IV) Benzylisocyanat, substituiertes Benzylisocyanat, Phenethylisocyanat, substituiertes Phenethylisocyanat, 3-Phenylpropylisocyanat oder substituiertes 3-Phenylpropylisocyanat ist; oder wobei R⁴ in der Verbindung der Formel (IV) Aryl ist; oder wobei die Verbindung der Formel (IV) Phenylisocyanat oder substituiertes Phenylisocyanat ist; oder wobei das substituierte Phenylisocyanat mit Halogen, C₁₋₆-Alkoxy oder C₁₋₆-Alkyl substituiert ist.

14. Zusammensetzung nach Anspruch 1, wobei die Zusammensetzung ferner ein zweites Therapeutikum umfasst, das ein Antikrebsarzneimittel ist; oder wobei die Zusammensetzung ferner ein zweites Therapeutikum umfasst, das SM88 ist; oder wobei die Zusammensetzung ferner ein zweites Therapeutikum umfasst, das eine oder mehrere Komponenten von SM88 ist.

15. Zusammensetzung nach Anspruch 1, wobei das Verhältnis der Verbindung der Formel (I) zu dem Amid der Formel (III) etwa 10:1 beträgt.

16. Zusammensetzung nach Anspruch 1, umfassend
Nonaethylenglycolmonododecylether;
1-Methyl-2-pyrrolidon;
Ethanol;
Linolsäure; und
ein Therapeutikum, das Allylisothiocyanat, 1-Isothiocyanato-4-methylsulfinylbutan, Benzylisothiocyanat, substituiertes Benzylisothiocyanat, Phenethylisothiocyanat, substituiertes Phenethylisothiocyanat, Benzylisocyanat, substituiertes Benzylisocyanat, Phenethylisocyanat oder substituiertes Phenethylisocyanat ist;
wobei die Zusammensetzung die transdermale Abgabe des Therapeutikums ermöglicht.

17. Zusammensetzung nach Anspruch 16, wobei die Zusammensetzung ferner ein zweites Therapeutikum umfasst, das ein Antikrebsmittel ist; oder ferner ein zweites Therapeutikum umfasst, das SM88 ist; oder ferner ein zweites Therapeutikum umfasst, das eine oder mehrere Komponenten von SM88 ist.

18. Zusammensetzung nach Anspruch 16, wobei das Verhältnis (v/v) des Nonaethylenglycolmonododecylethers zu dem 1-Methyl-2-pyrrolidon 9:1 bis 11:1 beträgt; oder wobei das Verhältnis (v/v) des Nonaethylenglykolmonododecylethers zu dem 1-Methyl-2-pyrrolidon etwa 10:1 beträgt.

19. Zusammensetzung nach einem der Ansprüche 1 bis 18 zur Verwendung bei der Behandlung oder Vorbeugung von Krebs bei einem Säugetier, wobei die Zusammensetzung auf die Haut oder Schleimhaut des Säugetiers für eine ausreichende Zeit aufgetragen wird, um die Permeation einer wirksamen Menge des Therapeutikums der Formel (IV) durch die Haut oder Schleimhaut zu erreichen.

## Revendications

1. Composition comprenant un agent thérapeutique de la formule (IV) :
R⁴-N=C=Z (IV)
dans laquelle Z est S ou O, et R⁴ est un alkyle en C₁₋₆, un alcényle en C₂₋₆, un alk-aryle en C₁₋₆ ou un aryle, et :
- un composé de la formule générale (I),
R-(OCH₂CH₂)_{y}-OH (I)
dans laquelle R est un alkyle en C₁₋₂₀ ou un alcényle en C₂₋₂₀ ; et y vaut 1 à 25 ;
- un amide de la formule (III)
R²-N(R¹)-C(O)-R³ (III)
dans laquelle chaque R¹ est indépendamment H ou alkyle en C₁₋₃ ; et R² et R³ ensemble avec les atomes auxquels ils sont liés forment un lactame ayant de 3 à 10 atomes de carbone ;
- un alcool d'alkyle en C₂₋₁₀ ;
- un acide organique qui est un acide gras, ayant de 1 à 25 atomes de carbone.

2. Composition selon la revendication 1, dans laquelle R est un alkyle en C₁₋₂₀ ; ou dans laquelle le composé de formule (I) est le cétomacrogol 1000 ; l'octadécane-1-ol éthoxylé ; l'éther tridécylique de polyoxyéthylène(12) ; l'éther tridécylique de polyoxyéthylène(10) ; l'éther de polyoxyéthylène d'alcool gras, l'éther nonylcyclohexylique ramifié de polyoxyéthylène, l'éther monododécylique de nonaéthylèneglycol, le 23-{[4-(2,4,4-triméthyl-2-pentanyl)cyclohexyl]oxy}-3,6,9,12,15,18,21-heptaoxatricosan-1-ol, ou une combinaison de ceux-ci ; ou dans laquelle le composé de formule (I) est l'éther monododécylique de nonaéthylèneglycol.

3. Composition selon la revendication 1, dans laquelle R est un alcényle en C₂₋₂₀ ; ou dans laquelle le composé de formule (I) est l'éther oléylique de polyoxyl(10), l'éther tert-octylphénylique de polyéthylèneglycol ou une combinaison de ceux-ci.

4. Composition selon la revendication 1, dans laquelle y vaut de 5 à 15 ; ou dans laquelle y vaut de 8 à 10 ; ou dans laquelle y vaut 9.

5. Composition selon la revendication 1, dans laquelle R¹ est méthyle, éthyle ou propyle.

6. Composition selon la revendication 1, dans laquelle le lactame est une pyrrolidone ; de préférence dans laquelle la pyrrolidone est la 2-pyrrolidone, la 1-méthyl-2-pyrrolidone, la 5-méthyl-2-pyrrolidone ou la 1-éthyl-2-pyrrolidone ; le plus préférablement dans laquelle la pyrrolidone est la 1-méthyl-2-pyrrolidone.

7. Composition selon la revendication 1, dans laquelle l'alcool d'alkyle en C₂₋₁₀ est le glycérol, le propylène glycol, l'éthanol, l'isopropanol, le 1-propanol, le butanol, le t-butanol, le pentanol, le 1-octanol ou une combinaison de ceux-ci ; le plus préférablement dans laquelle l'alcool d'alkyle en C₂₋₁₀ est l'éthanol.

8. Composition selon la revendication 1, dans laquelle l'acide gras est un acide gras saturé, un acide gras insaturé ou un acide gras polyinsaturé ; ou dans laquelle l'acide gras est l'acide butyrique, l'acide valérique, l'acide caproïque, l'acide énanthique, l'acide caprylique, l'acide pélargonique, l'acide caprique, l'acide undécylique, l'acide laurique, l'acide tridécylique, l'acide myristique, l'acide pentadécylique, l'acide palmitique, l'acide margarique, l'acide stéarique, l'acide isostéarique, l'acide nonadécylique, l'acide arachidique, l'acide hénéicosylique, l'acide béhénique, l'acide tricosylique, l'acide lignocérique, l'acide pentacosylique, l'acide cérotique, l'acide heptacosylique, l'acide montanique, l'acide nonacocylique, l'acide mélissique, l'acide hénatriacontylique, l'acide laccéroïque, l'acide psyllique, l'acide geddique, l'acide céroplastique ou l'acide hexatriacontylique ; ou dans laquelle l'acide gras est l'acide hexadécatriénoïque, l'acide alpha-linolénique, l'acide eicosapentanoïque, l'acide linoléique, l'acide docosadiénoïque, l'acide arachidonique, l'acide tétrasatétraénoïque, l'acide oléique, l'acide eicosénoïque, l'acide névronique, l'acide ruménique, l'acide éléostatique ou l'acide rumélénique ; ou dans laquelle l'acide gras est l'acide 14-méthylpentadécanoïque, l'acide 6-méthylcaprylique, l'acide 4-méthyl-3-penténoïque (acide pyrotérébique), l'acide 2-méthyl-2E-buténoïque (acide tiglique), l'acide 2-méthyl-2Z-buténoïque (acide angélique), la vittatalactone (acide tout-trans-rétinoïque), l'acide 2-hydroxyoctanoïque ou l'acide 4-oxopentanoïque.

9. Composition selon la revendication 1, dans laquelle Z dans le composé de formule (IV) est S ; ou dans laquelle R⁴ dans le composé de formule (IV) est un alkyle en C₁₋₆ ; ou dans laquelle le composé de formule (IV) est l'isothiocyanate de méthyle, l'isothiocyanate de méthyle substitué, l'isothiocyanate d'éthyle, l'isothiocyanate d'éthyle substitué, l'isothiocyanate de propyle, l'isothiocyanate de propyle substitué, l'isothiocyanate de butyle, l'isothiocyanate de t-butyle, l'isothiocyanate de butyle substitué, l'isothiocyanate de pentyle, l'isothiocyanate de pentyle substitué, les isothiocyanates d'hexyle ou les isothiocyanates d'hexyle substitués ; ou dans laquelle l'isothiocyanate de propyle est l'isothiocyanate d'isopropyle ; ou dans laquelle l'isothiocyanate de butyle est l'isothiocyanate de t-butyle ; ou dans laquelle l'isothiocyanate de butyle substitué est le 1-isothiocyanato-4-méthylsulfinylbutane.

10. Composition selon la revendication 9, dans laquelle R⁴ dans le composé de formule (IV) est un alcényle en C₂₋₆ ; ou dans laquelle le composé de formule (IV) est l'isothiocyanate d'éthényle, l'isothiocyanate d'éthényle substitué, l'isothiocyanate de propényle, l'isothiocyanate de propényle substitué, l'isothiocyanate de butényle, l'isothiocyanate de butényle substitué, l'isothiocyanate de pentényle, l'isothiocyanate de pentényle substitué, l'isothiocyanate d'hexényle, l'isothiocyanate d'hexényle substitué ; ou dans laquelle l'isothiocyanate de propényle est l'isothiocyanate d'allyle.

11. Composition selon la revendication 9, dans laquelle R⁴ dans le composé de formule (IV) est un alk-aryle en C₁₋₆ ; ou dans laquelle le composé de formule (IV) est l'isothiocyanate de benzyle, l'isothiocyanate de benzyle substitué, l'isothiocyanate de phénéthyle, l'isothiocyanate de phénéthyle substitué, l'isothiocyanate de 3-phénylpropyle ou l'isothiocyanate de 3-phénylpropyle substitué.

12. Composition selon la revendication 9, dans laquelle R⁴ dans le composé de formule (IV) est un aryle ; ou dans laquelle le composé de formule (IV) est l'isothiocyanate de phényle ou l'isothiocyanate de phényle substitué ; ou dans laquelle l'isothiocyanate de phényle substitué est substitué par halogène, alcoxy en C₁₋₆ ou alkyle en C₁₋₆.

13. Composition selon la revendication 1, dans laquelle Z dans le composé de formule (IV) est O ; ou dans laquelle R⁴ dans le composé de formule (IV) est un alkyle en C₁₋₆ ; ou dans laquelle le composé de formule IV est l'isocyanate de méthyle, l'isocyanate de méthyle substitué, l'isocyanate d'éthyle, l'isocyanate d'éthyle substitué, l'isocyanate de propyle, l'isocyanate de propyle substitué, l'isocyanate de butyle, l'isocyanate de butyle substitué, l'isocyanate de pentyle, l'isocyanate de pentyle substitué, l'isocyanate d'hexyle ou l'isocyanate d'hexyle substitué ; ou dans laquelle l'isocyanate de propyle est l'isocyanate d'isopropyle ; ou dans laquelle l'isocyanate de butyle est l'isocyanate de t-butyle ; ou dans laquelle R⁴ dans le composé de formule (IV) est un alcényle en C₂₋₆ ; ou dans laquelle le composé de formule (IV) est l'isocyanate d'éthényle, l'isocyanate d'éthényle substitué, l'isocyanate de propényle, l'isocyanate de propényle substitué, l'isocyanate de butényle, l'isocyanate de butényle substitué, l'isocyanate de pentényle, l'isocyanate de pentényle substitué, l'isocyanate d'hexényle ou l'isocyanate d'hexényle substitué ; ou dans laquelle l'isocyanate de propényle est l'isocyanate d'allyle ; ou dans laquelle R⁴ dans le composé de formule (IV) est un alk-aryle en C₁₋₆ ; ou dans laquelle le composé de formule (IV) est l'isocyanate de benzyle, l'isocyanate de benzyle substitué, l'isocyanate de phénéthyle, l'isocyanate de phénéthyle substitué, l'isocyanate de 3-phénylpropyle ou l'isocyanate de 3-phénylpropyle substitué ; ou dans laquelle R⁴ dans le composé de formule (IV) est aryle ; ou dans laquelle le composé de formule (IV) est l'isocyanate de phényle ou l'isocyanate de phényle substitué ; ou dans laquelle l'isocyanate de phényle substitué est substitué par halogène, alcoxy en C₁₋₆ ou alkyle en C₁₋₆.

14. Composition selon la revendication 1, dans laquelle la composition comprend en outre un second agent thérapeutique qui est un médicament anticancéreux ; ou dans laquelle la composition comprend en outre un second agent thérapeutique qui est le SM88 ; ou dans laquelle la composition comprend en outre un second agent thérapeutique qui est un ou plusieurs composants de SM88.

15. Composition selon la revendication 1, dans laquelle le rapport du composé de formule (I) à l'amide de formule (III) est d'environ 10:1.

16. Composition selon la revendication 1 comprenant éther
monododécylique de nonaéthylèneglycol ;
1-méthyl-2-pyrrolidone ;
éthanol ;
acide linoléique ; et
un agent thérapeutique qui est l'isothiocyanate d'allyle, le 1-isothiocyanato-4-méthylsulfinylbutane, l'isothiocyanate de benzyle, l'isothiocyanate de benzyle substitué, l'isothiocyanate de phénéthyle, l'isothiocyanate de phénéthyle substitué, l'isocyanate de benzyle, l'isocyanate de benzyle substitué, l'isocyanate de phénéthyle ou l'isocyanate de phénéthyle substitué ;
dans laquelle la composition permet l'administration transdermique de l'agent thérapeutique.

17. Composition selon la revendication 16, dans laquelle la composition comprend en outre un second agent thérapeutique qui est un médicament anticancéreux ; ou comprend en outre un second agent thérapeutique qui est le SM88 ; ou comprend en outre un second agent thérapeutique qui est un ou plusieurs composants de SM88.

18. Composition selon la revendication 16, dans laquelle le rapport (v/v) de l'éther monododécylique de nonaéthylèneglycol à la 1-méthyl-2-pyrrolidone est de 9:1 à 11:1 ; ou dans laquelle le rapport (v/v) de l'éther monododécylique de nonaéthylèneglycol à la 1-méthyl-2-pyrrolidone est d'environ 10:1.

19. Composition selon l'une quelconque des revendications 1 à 18 destinée à être utilisée dans le traitement ou la prévention du cancer chez un mammifère, dans laquelle la composition est appliquée sur la membrane cutanée ou muqueuse dudit mammifère pendant une durée suffisante pour obtenir la perméation d'une quantité efficace de l'agent thérapeutique de formule (IV) à travers la membrane cutanée ou muqueuse.
